# EUROPEAN PATENT SPECIFICATION

(11) **EP 4 124 330 B1**
(45) Date of publication and mention of the grant of the patent: **23.08.2023**
(21) Application number: 21195309.6
(22) Date of filing: 07.09.2021
(51) Int. Cl.: A61J 1/16, A61B 50/33, A61J 1/20

(54) **COMPONENT MIXING APPARATUS AND SYSTEM INCLUDING A CANNULA FOR FLUID TRANSFER**
KOMPONENTENMISCHVORRICHTUNG UND SYSTEM MIT EINER KANÜLE ZUR FLÜSSIGKEITSÜBERTRAGUNG
APPAREIL ET SYSTÈME DE MÉLANGE DE COMPOSANTS COMPRENANT UNE CANULE DE TRANSFERT DE FLUIDE

(30) Priority: 26.07.2021 IN 202121033506
(43) Date of publication of application: 01.02.2023
(73) Proprietor: Kairish Innotech Private Ltd., Mumbai 400021 (IN)
(72) Inventor: Dadachanji, Rishad Kairus, 400005 Mumbai (IN); Potdar, Pratul Prakash, 396210 Nani Daman (IN); Patel, Keyurkumar Arvindbhai, 396210 Daman, Daman & Diu (IN); Chudasma, Krupal Ashokbhai, 382345 Ahmedabad (IN)
(74) Representative: 2K Patentanwälte Blasberg Kewitz & Reichel

(56) References cited:
- WO-A2-00/25846
- FR-A1- 2 293 916
- JP-A- H0 549 672
- US-A- 5 445 631
- US-A1- 2016 296 420

## Description

### Field of Invention

The present invention generally relates to reconstitution of a drug solution or pharmaceutical solution for medical or pharmaceutical applications by combining a first liquid component and a second solid or liquid component by means of negative pressure, and relates more specifically to an apparatus for combining a first liquid component stored in a container and a second solid or liquid component stored in a medical vial by means of negative pressure. The present invention also relates to a system and packaging unit including such an apparatus, which stores or packages a vial and a container for the afore-mentioned purposes.

### Background of Invention

Certain drugs are preferably provided in powder or dry form (such as a lyophilized form), and require liquid reconstitution prior to administration to a patient. Lyophilized drugs, for example, typically are supplied in a freeze-dried form that must be mixed with a diluent to reconstitute the substance into a form that is suitable for injection.

Drugs may also be provided as multi-component systems which require mixing prior to administration. For example, a multi-component system may include one or more liquid (e.g., flowable) components and/or dry (e.g., powdered or granular) components which must be mixed prior to administration.

There are a number of devices and methods for drug reconstitution, where a lyophilized drug is stored and supplied in a standard vial under negative pressure.

According to a first approach, a user uses a first syringe to inject the diluent from a diluent vial into the vial containing the dry drug. Next the user withdraws the reconstituted drug from the vial using a second syringe. The second syringe is used to inject the reconstituted drug into a patient. The use of syringe with injection needles bears the risk of inadvertent injuries of users or patients.

A second approach avoids the use of syringes with injection needles by means of specially designed vial adapters that have a piercing mandrel for piercing or puncturing a vial stopper. For enabling an efficient needleless or needle-free coupling of a syringe with a vial, US 2017/0143586 A1 discloses an example for such a vial adapter comprising an outer thread for a Luer lock thread of a syringe for threading the syringe tip onto the vial adapter.

In medical applications, it is usually desirable to prevent the patient from being exposed to the fluid which is being injected to or extracted from the patient, and it is desirable to insulate nurses and doctors from exposure to the liquid which may contain the patient's blood or waste products.

Often the male component of the syringe used to inject or withdraw the fluid, retains some of the fluid on the tip thereof, thus providing a risk to nurses and doctors of being exposed to the fluid. Wiping off this fluid prior to disconnecting the syringe is highly desirable. For enabling a safe and efficient wiping off of fluid in such applications, US 6,651,956 B2 discloses a valve which includes a stem having a slit at an end thereof. The valve stem is located in a valve body and is deformable. When the tip of a syringe is engaged with the slit in the stem, the stem shifts in the valve body, a top portion thereof folds inward and the slit seals against the instrument and allows liquid to flow through the stem, to or from the instrument. When the tip of the syringe is removed again, the surface of the valve stem will not be contaminated with liquid.

Integrating such a valve stem into a vial adapter of the kind discussed above is also known from the prior art.

US 6,558,365 B2 discloses a third approach, which uses two vial adapters, namely a first adapter being connected to a container containing the powder component and a second adapter that can be removably interconnected with the first adapter and can also be readily connected to a container containing a fluid such as a diluent so as to permit aseptic intermixing of the diluent with the powder.

There is a continuous need for a reconstitution device with a safer and more accurate activation system which ensures that the needle mechanism used for injection a reconstituted drug solution will not be prematurely exposed or connected as it could lead to contamination of the drug and/or containers, loss of drug or safety risks.

There is also a need for a more user-friendly reconstitution device, which reduces the possibility of user error during the reconstitution process. A further need exists for an arrangement which would allow the device to be provided as a pre-assembled kit in which the vial and diluent container come pre-attached to reduce user handling steps; as a kit in which only the vial is attached; and/or as a separate system in which the vial and the diluent container are attached by a user just prior to use.

FR 2293916 A1 discloses an apparatus for combining a first liquid component stored in a container and a second solid or liquid component stored in a vial according to the preamble of claim 1. The accommodation member is a cylindrical oblong sleeve formed of plastic material, which is open at its two ends. A partition wall is arranged transversely inside the sleeve, so as to delimit two contiguous chambers intended to receive the vials. The separation wall has a slot in radial direction for insertion of a double perforating needle from a side of the sleeve for installation, and the slot ends in a slightly wider part in which the double perforating needle is intended to come to rest. After insertion of the two vials into the open ends of the sleeve, the vials are pushed towards the separation wall until the vial stoppers are pierced by the double perforating needle. The accommodation member is not formed as a tray having a planar upper surface.

US 20160296420 A1, US 5445631 A1 and JP 405049672A disclose similar sleeve-shaped transfer adaptors. WO 00/25846 A2 discloses a device for axial alignment of a syringe and a vial and guiding the axial sliding movement of the syringe towards the vial for self-administering of drugs.

### Summary of Invention

It is an object of the present invention to provide convenient, safe and reliable solutions for enabling reconstitution of a drug solution or pharmaceutical solution for medical or pharmaceutical applications by combining a first liquid component and a second solid or liquid component by means of negative pressure.

This problem is solved by an apparatus as further disclosed in claim 1, for combining a first liquid component stored in a container and a second solid or liquid component stored in a vial by means of negative pressure as claimed in claim 1 and by a system and packaging unit including such an apparatus as claimed in claim 14 and 15, respectively. Further advantageous embodiments are the subject-matter of the dependent claims.

According to the present invention there is provided an apparatus for combining a first liquid component stored in a container and a second solid or liquid component stored in a vial by means of negative pressure, comprising a tray having a vial cavity for accommodating at least a portion of the vial and a container cavity for accommodating at least a portion of the container, and a needle having a first needle tip and a second needle tip for establishing a fluid communication between the vial and the container in a transfer position, in which the first needle tip is engaged with the vial and the second needle tip is engaged with the container, wherein the needle is fixedly held at the tray at an intermediate needle holding portion between the vial cavity and the container cavity, wherein the vial cavity is configured for guiding a movement of the vial along an axial direction from an intermediate position, in which the first needle tip is not engaged with the vial, towards the transfer position, and wherein the container cavity is configured for guiding a movement of the container along the axial direction from an intermediate position, in which the second needle tip is not engaged with the container, towards the transfer position. According to the present invention the tray is having a planar upper surface, wherein the vial cavity and the container cavity is each open toward the planar upper surface and each formed for fully accommodating the vial and the container, respectively, in the intermediate position

The tray thus can be used as a jig, gauge or caliber to define the positional relationship between the vial and container in the intermediate or storage position and to guide them during the displacement from the intermediate position to the transfer position. From the intermediate position the transfer position can be accomplished by a simple axial displacement of the vial and diluent container, i.e., a displacement which is mainly in axial direction. In the transfer position, the two opposite needle ends of the transfer needle are engaged with the vial and diluent container, respectively, namely the rubber stopper of the vial and a bottom of the diluent container is each pierced by a needle end of the transfer needle, for enabling the transfer of liquid out of the diluent container and into the vial, e.g. for reconstitution of a liquid drug solution in the vial by mixing the second solid or liquid component stored in the vial with the diluent transferred from the container into the vial by the negative pressure prevailing inside the vial via the transfer needle.

The intermediate position may be identical with a storage position in which the vial and diluent container are stored in the tray over an extended period of time. According to further embodiments, however, the storage position is different to the intermediate position, and it is also conceived that the tray is not used for long-time storage of the vial and diluent container, but that the tray is used only for positioning the vial and diluent container relative to each other short time before effecting the axial displacement of the vial and diluent container from the intermediate position to the transfer position.

In the intermediate position the vial and diluent container may be positioned in axial alignment with each other, which shall mean that the axial center lines of the vial and diluent container, which are each generally of cylindrical shape, generally coincide. Of course, minor deviations from a perfect axial alignment between the vial and diluent container may exist in the intermediate position, and most important is that the axial displacement of the vial and diluent container is guided in such a manner that the needle ends start piercing or puncturing the vial stopper and container bottom, respectively, at its center and that the needle ends remain centered during the further displacement of the vial and diluent container from the intermediate position to the transfer position. The main purpose of the tray is thus to enable a sufficient centering effect so that the needle ends will pierce or puncture the vial stopper and container bottom at a central position.

'Maintaining the axial alignment of the vial and diluent container' during the transfer from the intermediate position to the transfer position, of course, shall allow a certain lateral displacement of the vial and diluent container, as long as the i.e., tray enables a sufficient centering effect so that the vial stopper and bottom of the diluent container will be pierced or punctured at a central position thereof.

The cavities of the tray member are preferably sufficiently deep so that the vial and diluent container each does not protrude beyond an upper surface of the tray, when the vial adapter and diluent container is accommodated inside the vial cavity and container cavity, respectively, so that e.g., a packaging foil may be bonded to the upper surface of the tray to provide a packaging unit. Both cavities are generally of cylindrical shape, preferably corresponding to the outer profile of the vial and diluent container, respectively. Preferably, the container cavity is large enough to fully accommodate the diluent container in the intermediate position, whereas a portion of the vial may extend beyond a front end of the vial cavity in the intermediate position, e.g., being exposed in an intermediate cavity between the vial cavity and needle packaging, for enabling access to the vial body with the fingers of a user or grippers of a robot to drive the axial displacement of the vial.

According to invention further embodiment, the vial cavity or the vial cavity and the container cavity may each comprise retaining members, which are configured to position the vial and the diluent container, respectively, in the intermediate position and in parallel with the axial direction of the cavities. The retaining members may each be formed simply by a bottom of the vial cavity and container cavity itself. The bottoms of the cavities then may serve for establishing an axial alignment between the vial and diluent container in the intermediate position and maintaining this axial alignment during the transfer from the intermediate position to the transfer position. During the transfer from the intermediate position to the transfer position, the bottoms of the cavities will then also be used for guiding the axial displacement of the vial and diluent container. Preferably, the bottom of at least the vial cavity is curved corresponding to the outer profile of the vial.

According to invention further embodiment, the retaining members may comprise pairs of protrusions formed on opposite side-walls of the vial cavity and container cavity, respectively, which are configured for contacting side-surfaces of the vial and container, respectively, for positioning the vial and the container in the respective cavity. The retaining members may be configured for preventing a displacement of the vial and diluent container in a direction perpendicular to the axial direction. Of course, the retaining members may also serve for guiding the axial displacement of the vial and diluent container, respectively, from the intermediate position to the transfer position.

According to a further embodiment, the retaining members may comprise pairs of protrusions formed on opposite side-walls of the vial cavity and container cavity, respectively, which are configured for contacting side-surfaces of the vial and diluent container, respectively, for positioning the vial and diluent container. By contacting side-surfaces of the vial and diluent container, respectively, the protrusions may control and keep constant the level of the vial and diluent container, respectively, in the cavities of the tray during the transfer from the intermediate position to the transfer position.

According to a further embodiment, a height of contact regions of the protrusions with the side-surfaces of the vial and diluent container, respectively, above a bottom of the vial cavity and container cavity, respectively, may be larger than the height of a center line of the vial and diluent container above the bottom of the vial cavity and container cavity. The protrusions may thus serve to delimit the level of the vial and diluent container, respectively, in a direction perpendicular to their axial direction, for preventing a significant lateral displacement of the vial and diluent container, respectively, in the direction perpendicular to their axial direction during the transfer from the intermediate position to the transfer position, to thereby maintain the alignment of the vial and diluent container and ensure a proper piercing of the vial stopper and container bottom, as outlined above.

According to a further embodiment, the opposite side-walls on which the protrusions are formed may each be upright and planar side-walls. Thus, the vial and diluent container can each be accommodated in cylindrical volumes formed between the bottom of the respective cavity and associated protrusion. Preferably, the upright and planar side-walls are a little flexible so that the protrusions can move a little outward when the vial and diluent container are inserted from above into the respective cavity. And preferably, the upright and planar side-walls will then flex back to their home position once the vial and diluent container has been inserted from above into the respective cavity, to secure the level of the vial and diluent container, respectively.

According to a further embodiment, the vial cavity may comprise at least two pairs of protrusions formed on opposite side-walls of the vial cavity, and at least one pair of protrusions may still be in contact with side-surfaces of the vial in the transfer position, when the first end of the needle pierces the rubber stopper of the vial. The at least one pair of protrusions may then be used for guiding the axial displacement of the vial toward the transfer position and maintaining the centering effect even at the final stage of the transfer from the intermediate position to the transfer position.

According to a further embodiment, a bottom of the vial cavity may be curved with a radius of curvature corresponding to an outer radius of a vial body of the vial, so that the bottom of the vial cavity can be used directly as a retaining member for the vial, as outlined above.

According to a further embodiment, the profile of a bottom of the container cavity may correspond to an outer profile of the diluent container, so that the bottom of the container cavity can be used directly as a retaining member for the container, as outlined above.

According to a further embodiment, the vial cavity may further comprise axial position limiting members configured for delimiting an axial movement of the vial inside the vial cavity in a storage position or in the intermediate position. The axial position of the vial can thus be defined precisely and in a simple manner, in order to ensure that the vial will be spaced apart from the first end of the transfer needle in the intermediate position. These axial position limiting members may be formed as protrusions on the bottom and/or side-walls of the vial cavity. Preferably, the axial position limiting members may be formed integrally with the bottom and/or side-walls of the vial cavity.

According to a further embodiment, the axial position limiting members are more flexible than the retaining members of the vial cavity. When the vial slides over the axial position limiting members during the transfer from the intermediate position to the transfer position, the axial position limiting members will thus not cause a significant displacement of the vial in a direction perpendicular to the axial direction of the vial.

According to a further embodiment, the vial cavity and the container cavity may each comprise a stop surface for delimiting an axial displacement of the vial and of the container by abutment with a front end of the vial and a bottom of the container, respectively. Thereby, the transfer position of the vial and diluent container can be precisely defined by abutment of the front end of the vial and bottom of the diluent container, respectively, with a bottom of the associated cavity of the tray.

According to a further embodiment, the vial cavity may further comprise a portion that is sufficiently wide to enable access to a vial body of the vial by means of fingers of a user or by means of grippers or the like of a robot in the intermediate position, for driving the axial displacement of the vial, and/or for removal of the vial from the tray member by means of fingers of a user or grippers of a robot.

According to a further embodiment, the tray member may further comprise a rear end cavity, where a bottom of the vial is sufficiently exposed to enable access to the bottom for a finger of a user or a manipulation member of a robot for driving the axial displacement of the vial from the intermediate position to the transfer position.

According to a further embodiment, the tray may comprise a tray member that is made of plastic material, in particular by vacuum thermoforming or pressure thermo-forming of a plastic sheet or by means of plastic injection molding. Or the tray member may be made of paper or cardboard with a thin film of plastic or bioplastic arranged on inner surfaces of the vial adapter cavity and vial cavity. Or the tray member is simply made of paper or cardboard without a thin film of plastic or bioplastic arranged on inner surfaces of the vial adapter cavity and vial cavity. Preferably, the retaining members are formed integrally with the tray member.

According to a further embodiment, the tray may comprise a first tray member having a vial cavity configured for long-time storage of the vial, preferably under sterile conditions, and a second tray member comprising the container cavity configured for long-time storage of the diluent container, preferably under sterile conditions.

The two tray members may be connected with each other to form the tray of said apparatus, so that both tray members may be stored or delivered separately, and connected with each other only shortly before performing a process for reconstitution of the drug solution.

According to a further embodiment, a central portion of the needle is accommodated in a needle packaging that is fixedly held at the tray at the intermediate needle holding portion. Moreover, the needle tips may be covered by cap members so that the needle can be packaged under sterile conditions, in particular also against the residual inner volume of the tray itself.

The needle tips may protrude into the vial cavity and container cavity, respectively, and the end caps may be configured for removal from the needle packaging or for being opened for access to the needle tips for fluid transfer.

According to a further embodiment, the needle packaging may be held at the tray at the intermediate needle holding portion in a positive-fit manner, by friction or adhesive bonding.

According to a further aspect of the present invention there is provided a system for combining a first liquid component stored in a container and a second solid or liquid component stored in a vial by means of negative pressure, comprising the apparatus as outlined above, wherein the vial is accommodated in the vial cavity of the tray at an intermediate position, in which the first needle tip is not engaged with the vial, towards the transfer position, and the container is accommodated in the container cavity at an intermediate position, in which the second needle tip is not engaged with the container, towards the transfer position.

According to a further embodiment, the needle is packaged under sterile conditions by a needle packaging.

According to a further aspect of the present invention there is provided a packaging unit for packaging a vial storing a second solid or liquid component together with a container storing a first liquid component, comprising a system as outlined in the previous paragraph, and a packaging foil, wherein the tray or at least the vial cavity is sealed against the environment by the packaging foil. The packaging unit may be used for sterile packaging the vial and diluent container.

As the tray comprise a planar upper surface, the packaging foil may be adhesively bonded to the upper surface of the tray.

### Overview on Drawings

The invention will now be described by way of example and with reference to the accompanying drawings, from which further features, advantages and problems to be solved will be-come apparent. In the drawings:
- Figs. 1a: shows an example of an apparatus for combining a first liquid component stored in a container and a second solid or liquid component stored in a vial by means of negative pressure according to the present invention in a perspective top view;
- Fig. 1b: shows a tray member vial portion of the apparatus of Fig. 1a in a top view;
- Fig. 1c: shows a tray member container portion of the apparatus of Fig. 1a in a top view;
- Fig. 1d: shows another example of a tray member container portion of the apparatus of Fig. 1a in a top view;
- Figs. 2a to 2d: show the tray member vial portion of an apparatus according to the present invention in a perspective top view, in a perspective bottom view, in a plan view and in a side-view;
- Fig. 2e: shows the tray member vial portion of Fig. 2a in a perspective top view, with the vial accommodated in the vial cavity of the tray member vial portion in a storage position that coincides with the intermediate position;
- Fig. 2f: shows the tray member vial portion of Fig. 2e in a perspective top view, with the vial displaced in axial direction towards the needle, shortly before the first end of the transfer needle pierces the vial stopper;
- Fig. 2g: shows a schematic cross-section of the tray member vial portion with the vial accommodated in the vial cavity, viewed towards the bottom of the vial;
- Fig. 3a: shows a needle packaging assembly for packaging a dual-ended transfer needle according to the present invention;
- Fig. 3b: shows another examiner of a needle packaging assembly according to the present invention;
- Fig. 4a: shows how the needle packaging assembly of Fig. 3b is disposed at the intermediate needle holding portion between the vial cavity and the container cavity, before removal of end caps of the needle packaging assembly;
- Fig. 4b: the needle packaging assembly of Fig. 4a after removal of end caps of the needle packaging assembly for providing access to the ends of the dual-ended needle for fluid transfer;
- Fig. 5a: shows a packaging unit for packaging a vial storing a second solid or liquid component together with a container storing a first liquid component in a schematic top view;
- Fig. 5b: shows tray of the packaging unit of Fig. 5a after removal of the packaging foil, with the vial and container disposed at the intermediate position;
- Figs. 5c to 5g: show the stages of a process for transferring a diluent stored in the container into the vial, for reconstituting a drug solution in the vial using an apparatus according to the present invention;
- Fig. 5h: shows the removal of the vial with the reconstituted drug solution after performing the steps of Figs. 5c to 5g; and
- Figs. 6a and 6b: show the apparatus according to the present invention with the vial and the container in the transfer position for fluid transfer in a top view and schematic cross-sectional view, respectively.

In the drawings, the same reference numerals designate identical or substantially equivalent elements or groups of elements.

### Detailed description of preferred embodiments

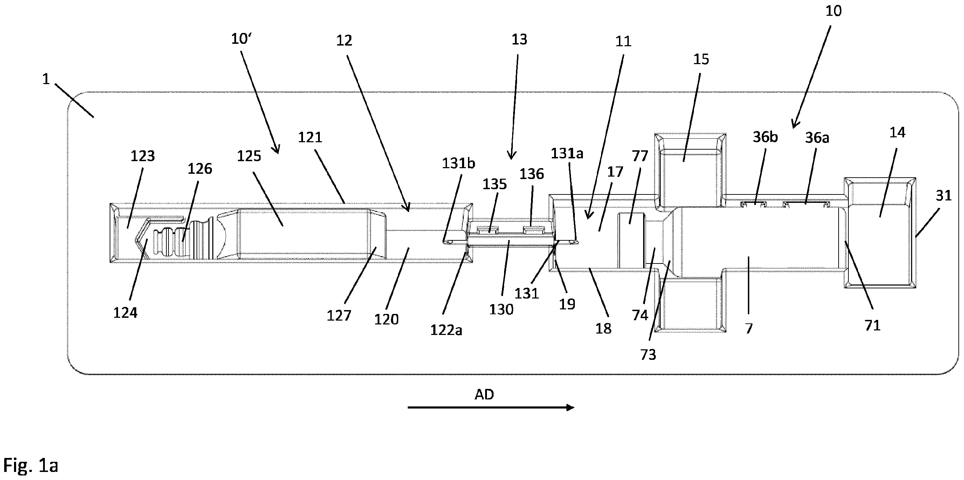

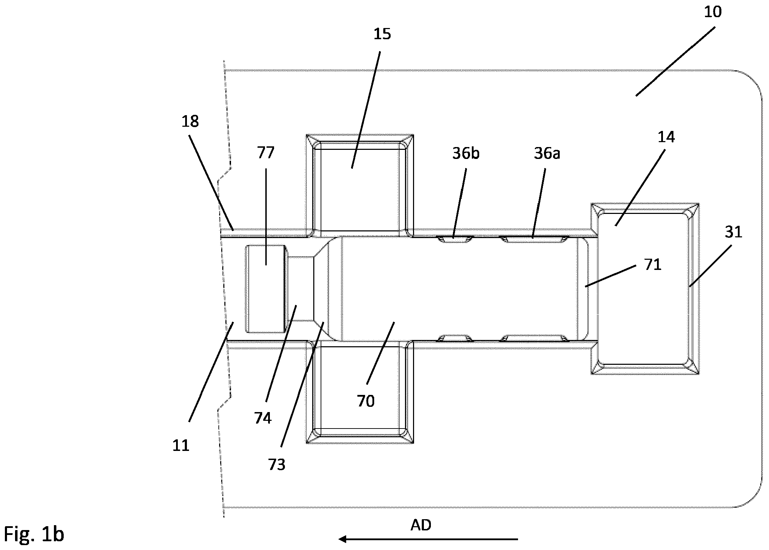

Fig. 1a shows an example of an apparatus for reconstitution of a drug solution by combining a first liquid component (e.g., diluent) stored in a container and a second solid or liquid component stored in a vial by means of negative pressure according to the present invention in a perspective top view. Figs. 1b to 1d show examples of a tray member vial portion 10 and of a tray member container portion 10' of such an apparatus.

The vial 7 can be made of glass or plastic material and is used to store a solid or liquid component under negative pressure. Preferably, the vial stores a lyophilized powder under negative pressure, which needs to be mixed with a diluent to reconstitute the substance into a form that is suitable for injection. The container 125 can be made of any material suited to store a diluent required for reconstitution of the drug solution and may also be embodied as a pouch made of a flexible foil. Preferably, the container 125 is of cylindrical or parallelepiped shape and is accommodated in a container cavity 12 of corresponding profile formed in the tray member 10. In particular, such a container 125 may be made of plastic material with rigid or semi-rigid side-walls.

The tray member vial portion 10 and the tray member container portion 10' are preferably each formed as a tray having a planar upper surface that can be sealed by adhesive bonding of a packaging foil, if required. The tray member vial portion 10 and the tray member container portion 10' are preferably formed integrally to a single tray 1 as shown in Fig. 1a, but may also be coupled together for liquid drug reconstitution, e.g., by means of positive-fit locking structures.

The tray member vial portion 10 comprises a vial cavity 11 that is open towards an upper surface of the tray member 10, will be described hereinafter in more detail and defines an axial direction AD. The vial cavity 11 is configured for accommodating a vial 7 and primarily serves as a jig, gauge or caliber for guiding an axial displacement of the vial 7 in the vial cavity 11 along the axial direction AD from an intermediate position, in which the first end 131a of the dual-ended needle 131 does not pierce a rubber stopper of the vial 7, towards a transfer position, in which the first end 131a of the dual-ended needle 131 pierces the rubber stopper of the vial 7 for fluid transfer. Of course, the vial cavity 13 may also be used for positioning the vial 7 in this intermediate position and preventing or at least reducing an axial displacement of the vial 7 from this intermediate position, in particular for reliably preventing the first end 131a of the dual-ended needle 131 piercing the rubber stopper of the vial 7. Of course, the vial cavity 13 may also be used for storage of the vial 7 in the intermediate position over an extended period of time. For this purpose, the vial cavity 13 may also be sealed against the environment by a packaging foil, as outlined below in more detail.

Correspondingly, the tray member container portion 10' comprises a container cavity 12 that is open towards the upper surface of the tray member 10', will be described hereinafter in more detail and defines an axial direction AD that is in parallel with the axial direction AD of the tray member vial portion 10 in the tray 1 shown in Fig. 1a. The container cavity 12 is configured for accommodating a diluent container 125 and primarily serves as a jig, gauge or caliber for guiding an axial displacement of the diluent container 125 in the container cavity 12 along the axial direction AD from an intermediate position, in which the second end 131b of the dual-ended needle 131 does not pierce a bottom 127 of the diluent container 125, towards a transfer position, in which the second end 131b of the dual-ended needle 131 pierces the a side-wall, in particular the bottom 127, of the diluent container 125 for fluid transfer. Of course, the container cavity 12 may also be used for positioning the diluent container 125 in this intermediate position and preventing or at least reducing an axial displacement of the diluent container 125 from this intermediate position, in particular for reliably preventing the second end 131b of the dual-ended needle 131 piercing the bottom of the diluent container 125. Of course, the container cavity 12 may also be used for storage of the diluent container 125 in the intermediate position over an extended period of time. For this purpose, the container cavity 12 may also be sterile sealed by a packaging foil, as outlined below in more detail.

As shown in Fig. 1a, a dual-ended needle 131 is fixedly held at the tray 1 at an intermediate needle holding portion 13 between the vial cavity 11 and the container cavity 12. For this purpose, the needle 131 is preferably packaged in a needle packaging 130 that prevents an axial displacement of the needle 131 inside the needle packaging 130 and that is fixed at an intermediate position between the vial cavity 11 and the container cavity 12. If the tray 1 is formed by two separate tray member portions 10, 10', which are coupled together for fluid transfer, the intermediate needle holding portion 13 is either provided at the front end of the vial cavity 11 or at the front end of the container cavity 12. Preferably, the needle 131 is packaged in the needle packaging 130 under sterile conditions against the residual volume of the tray 1.

As shown in Fig. 1a, the needle 131 is positioned at the center of the front wall 19 of the vial cavity 11 and at the center of the front wall 122a of the container cavity 12. The front wall 19 of the vial cavity 11 delimits the axial displacement of the vial 7 inside the vial cavity 11 towards the transfer position by abutment with the front end of the vial 7, in which the first end 131a of the needle 131 pierces the rubber stopper of the vial 7, whereas the front wall 122a of the container cavity 12 delimits the axial displacement of the diluent container 125 towards the transfer position by abutment with the bottom 127 of the diluent container 125, in which the second end of the needle 131 pierces the bottom 127 of the diluent container 125.

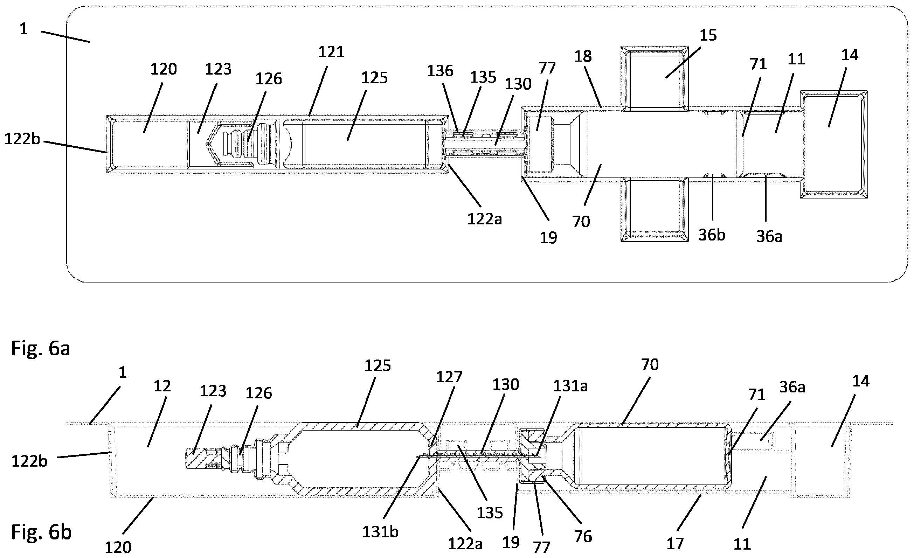

The general shape of a vial to be accommodated in the vial cavity 11 of a tray member vial portion 10 of the tray 1 is shown in Figs. 1b and 6b. The vial 7 has a cylindrical vial body 70 with a closed bottom 71 and a conical shoulder 73 that is followed by a narrow neck 74 and a wider rolled edge 75 that defines a filling opening of the vial 7. This filling opening is sealed by an elastomeric stopper 76 that is held in place by a cylindrical metal cap 77 that is crimped over the rolled edge 75. A circular central opening is defined in the upper surface of the metal cap 77 and exposes a central portion of the stopper 76 that will be pierced or punctured by the first end 131a of the dual-ended needle 131 in the transfer position shown in Figs. 6a and 6b, when the front end of the vial 7 abuts against the front wall of the vial cavity 11. The cylindrical shape of such a vial 7 precisely defines a center line.

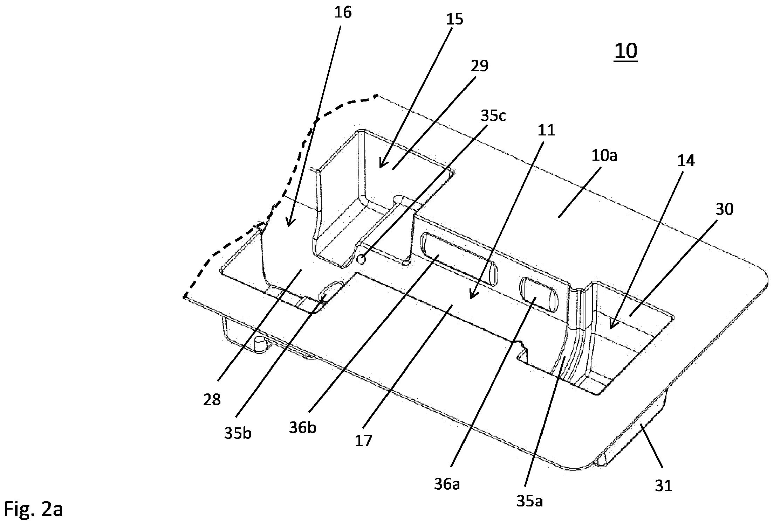

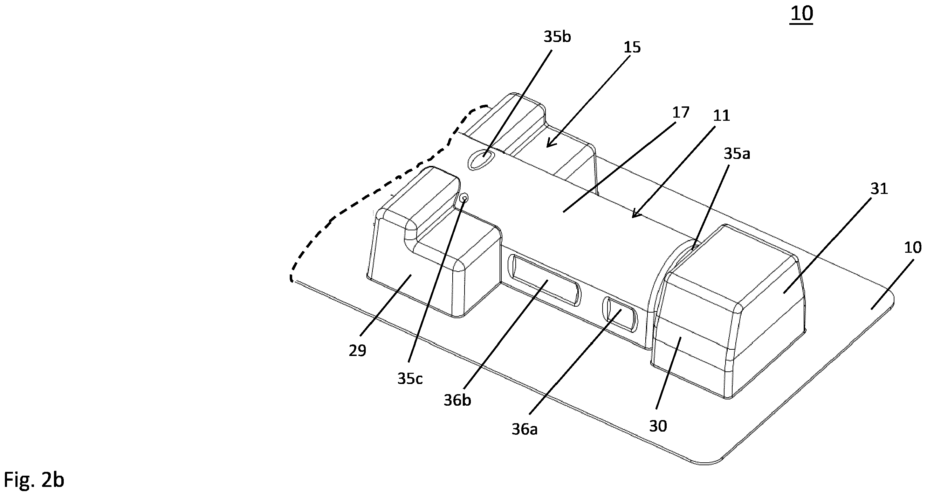

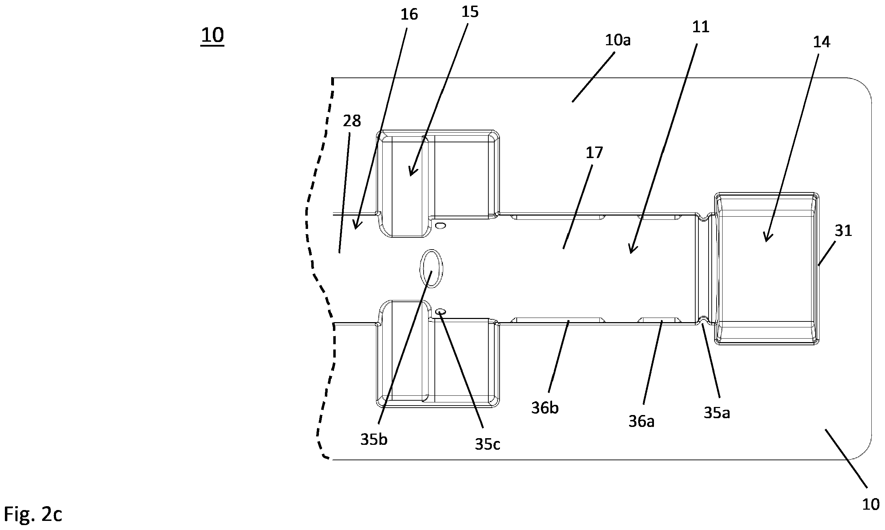

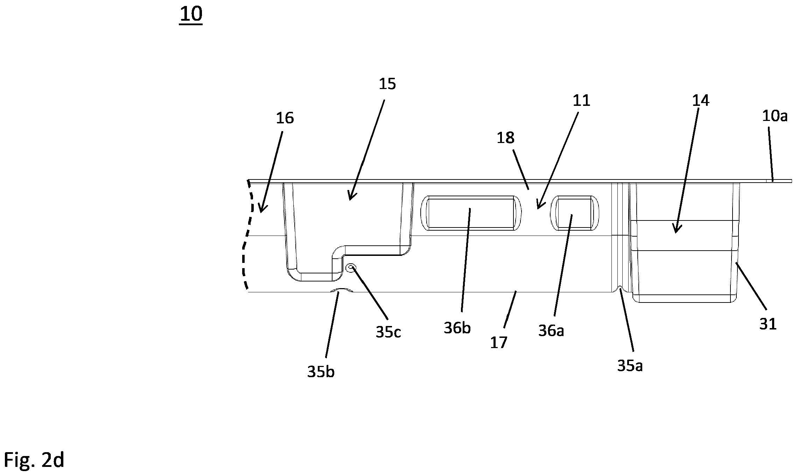

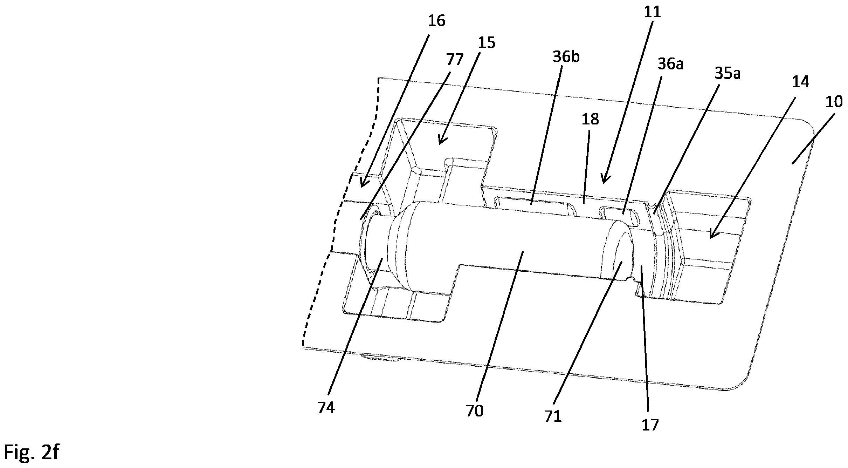

More details of the tray member vial portion 10 are shown in Figs. 2a-2g and will be explained in the following. The tray member vial portion 10 comprises a vial cavity 11 for accommodating a vial 7 spaced apart from the first end 131a of the dual-ended needle 131 and in axial alignment with the needle 131 in the intermediate position. The vial cavity 11 may have an inner profile corresponding to the outer contour of the vial body 70 (cf. Fig. 6b) of the vial 7 to be accommodated in the vial cavity 11. More specifically, the vial cavity 11 may have a curved bottom 17 having a radius of curvature that corresponds to the outer radius of the vial body 70. As shown in Figs. 2a and 2d, the two opposite upper side-walls 18 of the vial cavity 11 may be planar and extend perpendicular to the upper surface 10a of the tray member vial portion 10. As shown in Figs. 2a and 2d, a pair of front vial retaining members 36b is formed on the opposite upper side-walls 18 of the vial cavity 11 to retain the vial in the vial cavity 11 in the intermediate position. The front vial retaining members 36b may also serve to prevent a displacement of the vial 7 in a direction perpendicular to the axial direction of the vial cavity 11 and even to push the vial body 70 downward toward the bottom 17 of the vial cavity 11 to define the height of the center line CL of the vial 7 in the intermediate position. Additionally, a second pair of rear vial retaining members 36a may be formed on the opposite upper side-walls 18 of the vial cavity 11 to retain the vial 7 in the vial cavity 11 in the intermediate position. The rear vial retaining members 36a may also serve to prevent a displacement of the vial 7 in a direction perpendicular to the axial direction of the vial cavity 11 and even to push the vial body 70 downward toward the bottom 17 of the vial cavity 11 to define the height of the center line CL of the vial 7 in the intermediate position.

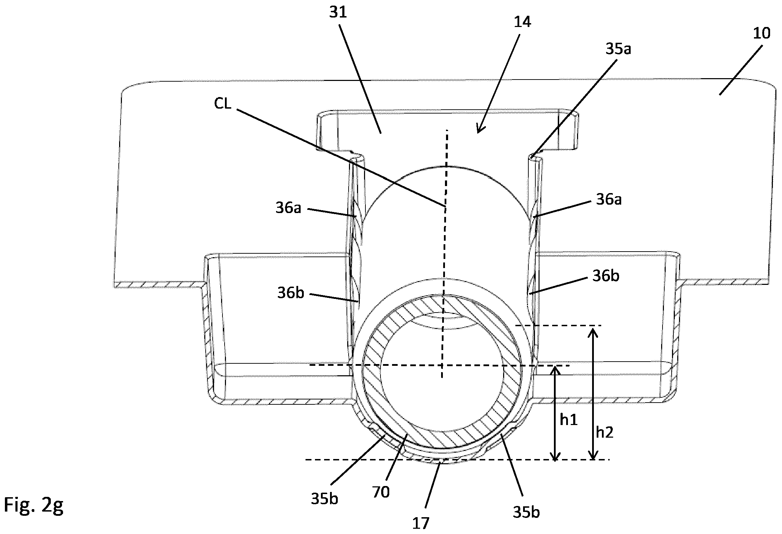

As shown in Fig. 2g, the retaining members 36a, 36b are preferably formed integrally with the upper side-walls 18 of the vial cavity 11. More specifically, the retaining members 36a, 36b may be formed as convexly curved protrusions protruding from the upper side-walls 18 of the vial cavity 11 at a height h2 that is larger than the height h1 of a center line CL of the vial body 70 above the bottom 17 of the vial cavity 11. Thus, a certain force component may always prevail to push the vial body 70 towards the bottom 17 of the vial cavity 11 when accommodated therein in the intermediate position. When a vial 7 is inserted from above into a vial cavity 11 for storage or positioning, the vial 7 will be locked by the retaining members 36a, 36b in the vial cavity 11 at least in a direction perpendicular to the center line CL of the vial body 70, to thereby define an orientation of the vial 7 in parallel with the bottom 17 of the vial cavity 11 and in axial alignment with the dual-ended needle 131. As shown in Fig. 2g, when the vial body 70 is accommodated in the vial cavity 11, it may not protrude beyond the upper surface 10a of the tray member vial portion 10 so that the vial cavity 11 may be sealed by a packaging foil bonded on the upper surface 10a of the tray member vial portion 10. Locking of the vial body 70 by the retaining members 36a, 36b in the vial cavity 11 may also be sufficient to define the position of the vial in axial direction.

As will become apparent to the skilled person, the bottom 17 and upper side-walls 18 of the vial cavity 11 may also embrace the vial body 70 by a little more than 180 degrees and thus serve directly as retaining members to position the vial 7 in the intermediate position and in parallel with the axial direction AD. For insertion of the vial 7 into the vial cavity 11 it would thus be necessary to flex the tray 1 a little about the central axis of the vial cavity 11 to open the vial cavity 11 a little bit more.

The vial body 70 may be clamped a little by the bottom 17 and upper side-walls 18 of the vial cavity 11 to define the position of the vial 7 in axial direction. As shown in Fig. 2d, movement limiting protrusions 35a, 35b may be provided in the vial cavity 11 near the rear end of the vial cavity 11 and near the position of the transition between the vial body 70 and the vial shoulder 73 (see Fig. 6b), for defining the position of the vial 7 in axial direction even more precisely by abutment of protrusions in the vial cavity 11 with the bottom 71 and shoulder 73 of the vial 7, respectively. Moreover, additional movement limiting protrusions 35c may be provided on the side surfaces of the vial cavity 11, in particular at the front end thereof.

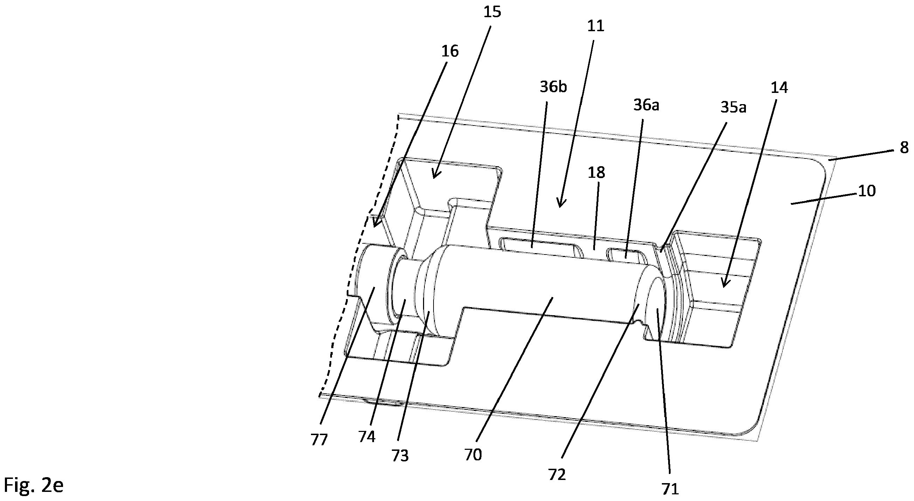

The vial body 70 may be accommodated in the vial cavity 11 in the intermediate position with a certain play in axial direction, but the vial body 70 may also be accommodated in the vial cavity 11 in the intermediate position without play in axial direction. The movement limiting protrusions 35a, 35b and 35c may be formed integrally with the bottom or side-walls of the vial cavity 11, and are preferably formed in the bottom 17 of the vial cavity 11, as shown in Fig. 2d. The movement limiting protrusions 35a, 35b, 35c may be formed as convex bulges protruding a little into the vial cavity 11. The rear movement limiting protrusion 35a may be U-shaped to extend along the entire rear end of the vial cavity 11, as shown in Fig. 2a. As the vial body 70 will slide over the front movement limiting protrusion 35b on its way towards the transfer position, the front movement limiting protrusion 35b may be relatively shallow and thin so that it can be pressed down easily by the vial body 70. As shown in Figs. 2a, 2d and 2e, the front movement limiting protrusion 35b may be disposed in the region of an intermediate cavity 16, which received the metal cap 77 / front end of the vial 7 in the intermediate position and through which the front end of the vial 7 when being displaced from the intermediate position toward the transfer position.

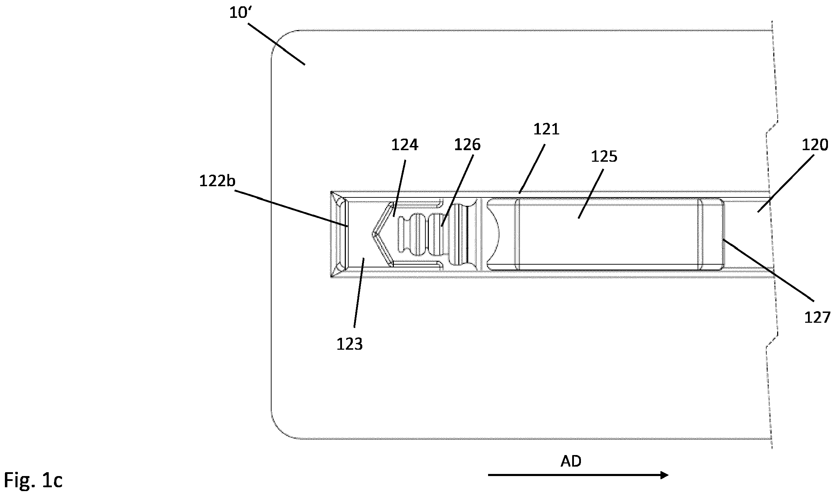

As shown in Figs. 1a and 1c, the tray member container portion 10' comprises a container cavity 12 for accommodating a diluent container 125 spaced apart from the second end 131b of the dual-ended needle 131 and preferably in axial alignment with the needle 131 in the intermediate position. The container cavity 12 may have an inner profile corresponding to the outer contour of the diluent container 12 to be accommodated in the container cavity 12. More specifically, the container cavity 12 may be formed by a planar bottom 120 and two opposite side-walls 121 which may be planar and extend perpendicular to the bottom 120 of the container cavity 12. As shown in Fig. 1c, the diluent container 125, at its end facing the rear wall 122b of the container cavity 12, may be closed by a closure 126 that seals a filling opening for filling the container 125 with a diluent. The closure 126 is received in a recess 124 of a pusher 123 that is slidably received in the container cavity 12 and serves for pushing the container 125 from the intermediate position shown in Figs. 1a and 1c toward the transfer position.

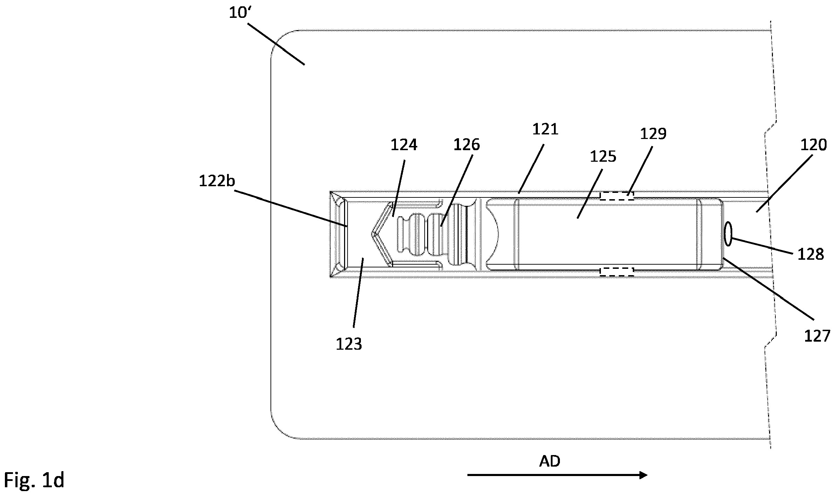

As shown in Fig. 1d, a pair of container retaining members 129 of similar configuration as outlined above for the vial retaining protrusions, may be formed on the opposite side-walls 121 of the container cavity 12 to retain the diluent container 125 in the container cavity 12 in the intermediate position. The container retaining members 129 may also serve to prevent a displacement of the container 125 in a direction perpendicular to the axial direction AD of the container cavity 12 and even to push the container 125 downward toward the bottom 120 of the container cavity 12 to define the height of the center line CL of the container 12 in the intermediate position.

As will become apparent to the skilled person, the bottom 120 and upper side-walls 121 of the container cavity 12 may also embrace the container 125 by a little more than 180 degrees and thus serve directly as retaining members to position the container 125 in the intermediate position and in parallel with the axial direction AD. For insertion of the container 125 into the container cavity 12 it would thus be necessary to flex the tray 1 a little about the central axis of the container cavity 12 to open the container cavity 12 a little bit more.

As shown in Fig. 1d, a movement limiting protrusion 128 may be provided in the container cavity 12 in front of the diluent container 125 in the intermediate position, for defining the position of the diluent container 12 in axial direction even more precisely by abutment of protrusion 128 in the container cavity 12 with the bottom 127 of the diluent container 125. Moreover, additional movement limiting protrusions may be provided on the side-walls 121 of the container cavity 12, in particular at the front end thereof.

The diluent container 125 may be accommodated in the container cavity 12 in the intermediate position with a certain play in axial direction, but the diluent container 125 may also be accommodated in the container cavity 12 in the intermediate position without play in axial direction. The movement limiting protrusion 128 may be formed integrally with the bottom 120 or side-walls 121 of the container cavity 12. The movement limiting protrusion 128 and the container retaining protrusions 129 may be formed as convex bulges protruding a little into the container cavity 12.

The tray 1 is preferably made of plastic material, in particular by vacuum thermoforming or pressure thermo-forming of a thin plastic sheet or by means of plastic injection molding, and preferably all of the retaining and movement limiting members 35a, 35b, 35c, 36a, 36b, 128 and 129 are formed integrally with the tray 1. Any other materials may be used as well, however. In particular, the tray 1 may also be made of paper or cardboard. A thin film of plastic or bioplastic may be arranged on inner surfaces of the diluent container cavity 12 and vial cavity 11 to enable even the storage of the diluent container and vial in the cavities 11, 12 under sterile conditions. DE 102011122211 A1 discloses an example of such a compound packaging material including a substrate made of paper or cardboard that is coated by a thin film of plastic or bioplastic. Preferably, the tray 1 may be a little flexible, so that it can be flexed a little about the middle axis of the cavities 11, 12, e.g., to ease insertion of the vial 7 and diluent container 125 into the cavities 11, 12, as outlined above.

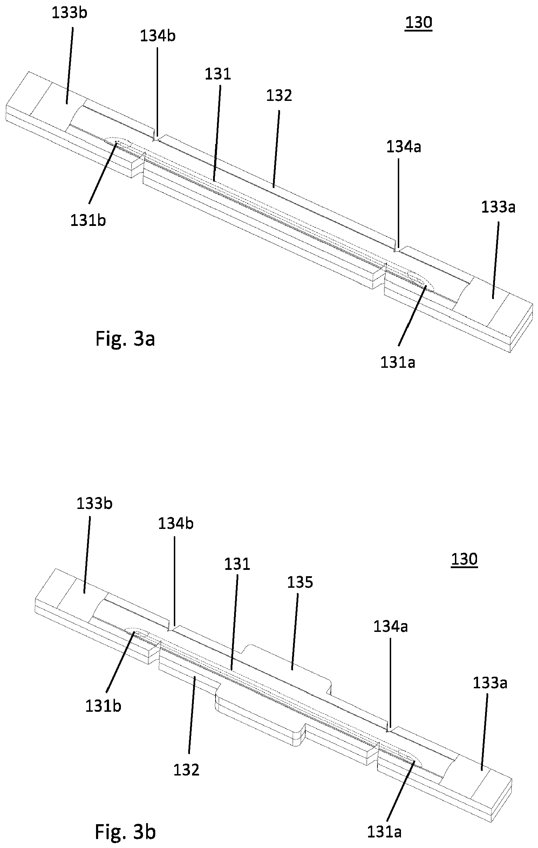

Fig. 3a shows further details of the needle packaging 130. The dual-ended needle 131 may be formed of plastic material, but may also be formed of a metal, such as stainless steel. The needle 131 is firmly received in the needle packaging 130 so that it does not move in axial direction, when the first and second end 131a, 131b get in contact with the vial stopper and bottom 127 of the diluent container 125, respectively. The needle packaging 130 may be a plastic foil tightly sleeved around the needle 131 or may be a block of plastic material which the needle 131 extends through. The first and second end 131a, 131b is each covered by an end cap member 133a, 133b, which is preferably integrally formed with the needle packaging 130 and may be torn or sheared off from the needle packaging 130. To ease removal of the end cap members 133a, 133b for providing access to the needle ends 131a, 131b, weakening zones or frangible portions 134a, 134b may be provided between the needle packaging 130 and the end cap members 1331, 133b. In particular, these weakening zones or frangible portions 134a, 134b may be notch-shaped, as shown in Fig. 3a.

More preferably, the needle 131 is received in the needle packaging 130 under sterile conditions against the residual volume of the cavities 11, 12 of the tray 1.

As shown in Fig. 1a, the needle packaging 130 is fixedly mounted to the tray 1 between the vial cavity 11 and the container cavity 12, so that the needle ends 131a, 131b protrude into the vial cavity 11 and container cavity 12, respectively. For this purpose, the needle packaging 130 may be held by friction at the tray 1, e.g., by clamping by means of deformable clamping brackets or by crimping. The needle packaging 130 may also be adhesively bonded to the tray 1 or may be bonded to the tray 1 by ultrasonic welding or applying heat to the needle packaging 130 and central part of the tray 1.

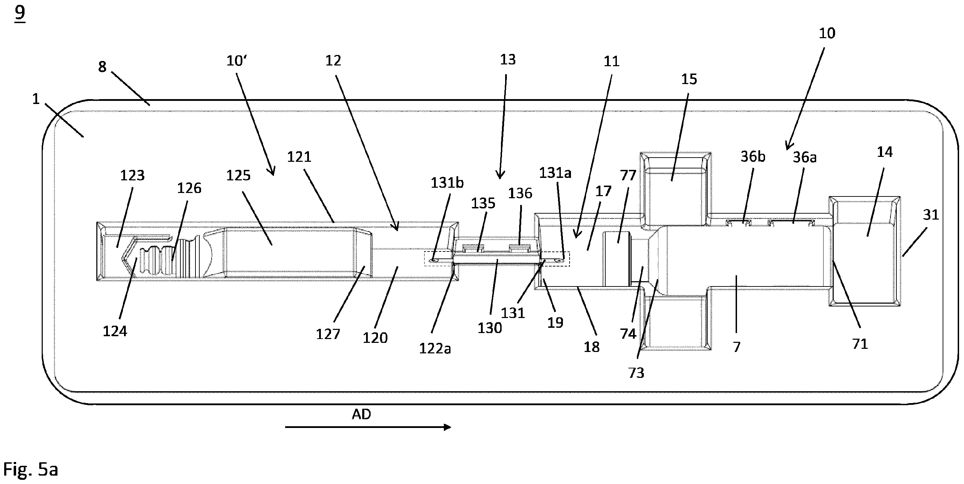

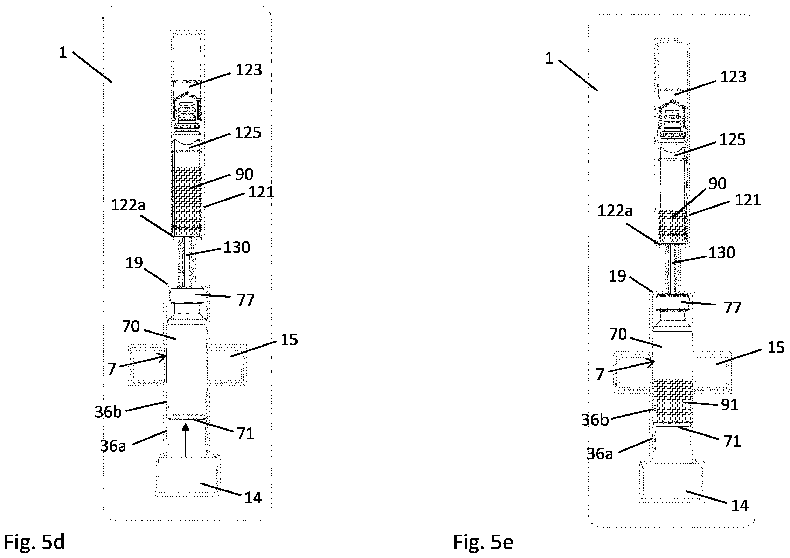

As shown in Fig. 3b, the needle packaging 130 may also be held by positive-fit at the tray 1. For this purpose, protrusions 135 may be formed at lateral side-walls of the needle packaging 130 that are received with positive-fit in corresponding recesses 136 formed on the tray 1, as shown in Fig. 5a. Or, recesses may be formed at lateral side-walls of the needle packaging 130 that engage with corresponding protrusions formed on the tray 1 in a positive-fit manner. Such protrusions or recesses may also be formed directly on the outer surface of the needle 131 or on extensions fixed mounted on the outer surface of the needle 131.

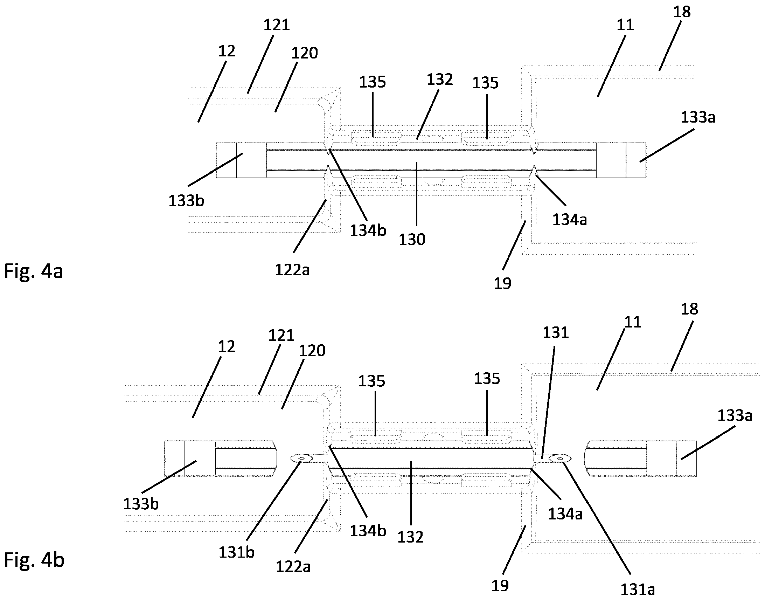

In the state shown in Fig. 4a, the end cap members 133a, 133b may seal the ends 131a, 131b of the needle 131 sterile against the local environment. Once, the end cap members 133a, 133b are removed, as shown in Fig. 4b, access to the needle ends 131a, 131b is permitted.

A system for combining a first liquid component stored in a container and a second solid or liquid component stored in a vial by means of negative pressure according to the present invention comprises the tray 1 with the needle 131 fixedly held at the tray 1 at an intermediate needle holding portion 13 between the vial cavity 11 and the container cavity 12, as shown in Fig. 1a, wherein the vial cavity 11 accommodates a vial 7 in an intermediate position, in which the first needle tip 131a is not engaged with the vial 7, and wherein the container cavity 12 accommodates a diluent container 12 in an intermediate position, in which the second needle tip 131b is not engaged with the container 125.

Fig. 5a shows a packaging unit 9 according to the present invention, consisting of such a system for combining a first liquid component stored in a container and a second solid or liquid component stored in a vial by means of negative pressure, which is sealed by a packaging foil 8. The packaging foil 8 may seal the whole tray 1 together with the vial 7 and diluent container 125 against the environment, and may seal the whole tray 1 in a sterile manner. For this purpose, the packaging foil 8 may be adhesively bonded onto the planar upper surface of the tray 1. As an alternative the tray 1 may be accommodated in a sealed pouch formed by the packaging foil 8. The packaging foil 8 may be gas-permeable, in particular a Tyvek^{®}-foil, to enable a steam sterilization of the tray 1, vial 7 and container 125 by a gas flowing through the packaging foil 8.

From the intermediate position shown in Fig. 5a, the transfer position shown in Figs. 6a and 6b, in which the first needle end 131a pierces the rubber stopper 76 of the vial 7 and in which the second needle end 131b pierces the bottom 127 of the diluent container 125, may be established by an axial displacement of the vial 7 towards the front wall 19 of the vial cavity 11 and by an axial displacement of the container 125 towards the front wall 122a of the container cavity 12.

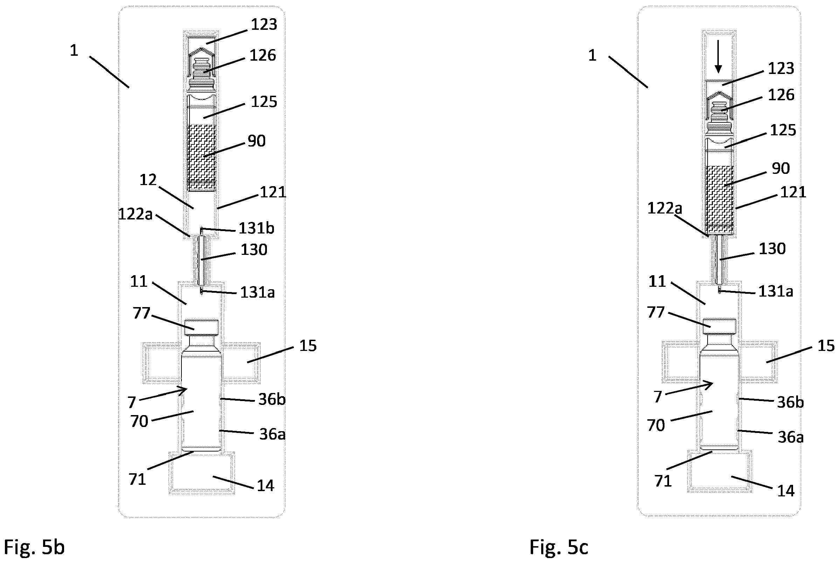

Starting with the packaging unit 9 shown in Fig. 5a, firstly the packaging foil 8 needs to be removed from the tray 1. Next, for drug reconstitution the steps shown in Figs. 5b to 5h are performed, as described below. These steps are preferably performed with the tray 1 held horizontally.

Fig. 5b shows the packaging unit after removal of the packaging foil 8. In this intermediate position, the vial 7 is spaced apart from the first needle end 131a and the container 125 is spaced apart from the second needle end 131b. The vial 7 stores e.g., a lyophilized drug component under negative pressure, whereas the container 125 stores a diluent 90, which is required for drug reconstitution.

As a next step, by pushing the pusher 123, the container 125 is pushed in the direction of the needle packaging 130 until the second needle end 131b engages the container 125 and pierces the bottom 127 or a side-wall of the container 125. For this purpose, the end cap member 133b (cf. Fig. 4a) may be removed only shortly before performing this step for providing access to the second needle end 131b. The axial displacement of the container 125 is guided by the bottom 120 and lateral side-walls 121 of the container cavity 12. Finally, the bottom of the container 125 will abut against the front wall 122a of the container cavity. This position of the container 125 may be fixed by fixing the position of the pusher 123 in the container cavity 12 by friction or positive-fit. As an example, the pusher 123 may be formed as a resilient, U-shaped member which is clamped against the side-walls 121 of the container cavity 12. As an alternative, a positive-fit structure, such as protrusions on the side-walls 121 of the container cavity 12, may be provided for positive-fit with the pusher 123 in the position shown in Fig. 5c.

As a next step, the vial 7 is pushed towards the first needle end 131a. To provide access to the first needle end 131a, the end cap member 133a (cf. Fig. 4a) may be removed only shortly before performing this step. The vial 7 is pushed in the direction of the needle packaging 130 until the first needle end 131a engages the vial 7 and pierces the rubber stopper of the vial 7. The axial displacement of the vial 7 is guided by the bottom and lateral side-walls of the vial cavity 11, and may be additionally guided by the retaining members 36a, 36b of the vial cavity 11. For driving the axial displacement of the vial 7, a user's finger or a pusher member of a robot may push against the bottom 71 of the vial 7 via the rear end cavity 14 of the tray 1 and/or fingers of a user or grippers of a robot may clamp the vial body 70 of the vial 7 via the widened lateral cavity 15, as outlined above.

Finally, the metal cap at the front end of the vial 7, which retains the rubber stopper, will abut against the front wall 19 of the vial cavity 11, and the first needle end 131a engages the vial 7 by piercing the rubber stopper. This transfer position of the vial 7 may be fixed by friction or positive-fit. As an example, the front retaining protrusions 36b may fix the transfer position of the vial 7 shown in Fig. 5d.

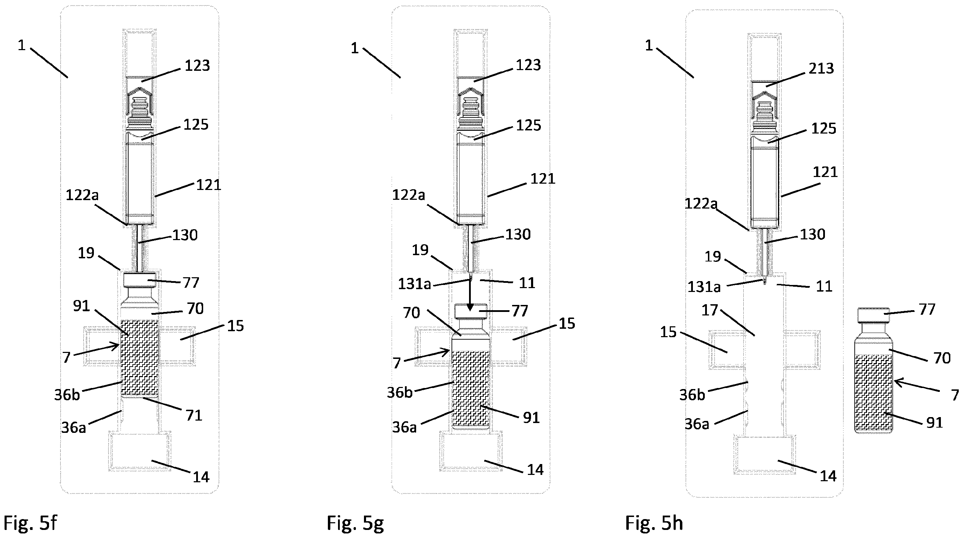

In the transfer position of Fig. 5d a fluid communication between the container 125 and the vial 7 has been established. The negative pressure in the vial 7 will drive the diluent 90 out of the container 125 and into the vial 7, until a pressure equilibrium is accomplished and at least the main portion of the diluent 90 has been transferred into the vial 7, as shown in Fig. 5f.

As a next step, the vial 7 is withdrawn from the front wall 19 of the vial cavity 11, to stop engagement with the first needle end 131a. The axial displacement of the vial 7 in the opposite direction is again guided by the bottom and lateral side-walls of the vial cavity 11, and may be guided additionally by the retaining members 36a, 36b of the vial cavity 11. For driving the axial displacement of the vial 7 in the opposite direction, a user's finger or a pusher member of a robot may push against the bottom 71 of the vial 7 via the rear end cavity 14 of the tray 1 and/or fingers of a user or grippers of a robot may clamp the vial body 70 of the vial 7 via the widened lateral cavity 15, as outlined above. Finally, the vial 7 reaches again the intermediate position shown in Fig. 5g, or a position near this intermediate position, in which the first needle end 131 does not engage the vial 7 anymore.

As a next step, the vial 7 is removed from the vial cavity 11, as shown in Fig. 5h, which leaves behind the tray 1 with the container 125 still in engagement with the second needle end 131b as well as the vial 7 including the reconstituted drug solution 91, which is then ready for use. As a final step, the needle of a syringe (not shown) may pierce the rubber stopper of the vial 7 for extraction of the reconstituted drug solution 91 from the vial 7.

Figs. 6a and 6b show the apparatus according to the present invention with the tray 1 and the vial 7 and the container 125 in the transfer position for fluid transfer in a top view and schematic cross-sectional view, respectively.

As will become apparent to the skilled person from the above description, the tray 1 is used as a jig, gauge or caliber to define the positional relationship between the vial and vial adapter in the intermediate position and to keep them in axial alignment during the axial displacement from the intermediate position to the transfer position. From the intermediate position the transfer position can be accomplished by a simple axial displacement of the vial and diluent container, i.e., a displacement only in axial direction. In the transfer position, the vial and container are in engagement with the needle ends for enabling the transfer of the liquid diluent out of the container and into the vial, e.g., for reconstitution of a liquid drug solution in the vial.

Of course, the tray 1 may also be used for long-time storage of the diluent container and vial, in particular under sterile conditions. For this purpose, a sterile packaging foil 8 may be adhesively bonded to the upper surface of the tray 1, as shown in Fig. 5a. Also, the needle ends 131a, 131b of the needle may be sterile sealed against the inner volume of the tray 1 and in particular against the vial cavity 11 and container cavity 12, in particular by means of the end cap members 133a, 133b of the needle packaging 130, as shown in Figs. 5a and 4a.

As will become apparent to the skilled person when studying the above description, a tray according to the present invention may be used for the storage / positioning / guiding of any kind of vial and diluent container. As a preferred example, the vial may store a lyophilized drug component under negative pressure. Of course, the vial may also store other drug components, such as dry powders required for drug reconstitution.

As will become apparent to the skilled person, also the diluent container 125 may be vial made of glass or plastic material, having a pierceable member, such as a rubber stopper, that seals the diluent container 125 and will be pierced by the second end 131b of the needle 131 in the afore-mentioned transfer position.

### List of reference numerals

- 1: tray

- 7: vial
- 8: foil
- 9: combined packaging unit

- 10: tray member portion for vial
- 10a: upper surface of tray member portion 10
- 10': tray member portion for diluent container
- 10a': upper surface of tray member portion 10'

- 11: vial cavity
- 12: diluent container cavity
- 13: needle holding portion

- 14: rear end cavity
- 15: lateral cavity
- 16: intermediate cavity

- 17: bottom of vial cavity 11
- 18: upper side-wall of vial cavity 11
- 19: front wall of vial cavity 11

- 28: bottom of intermediate cavity 16
- 29: side-wall of lateral cavity 15

- 35a: rear movement limiting protrusion
- 35b: front movement limiting protrusion
- 35c: front movement limiting protrusion
- 36a: rear vial retaining protrusion
- 36b: front vial retaining protrusion
- 70: vial body
- 71: vial bottom

- 73: shoulder
- 74: neck
- 75: rolled edge of vial
- 76: rubber stopper
- 77: metal cap

- 80: contact region

- 90: diluent
- 91: reconstituted drug

- 120: bottom of diluent container cavity 12
- 121: side-wall of diluent container cavity 12
- 122a: front wall of diluent container cavity 12
- 122b: rear wall of diluent container cavity 12
- 123: pusher
- 124: recess of pusher 123
- 125: diluent container
- 126: closure of diluent container 125
- 127: bottom of diluent container 125
- 128: movement limiting protrusion
- 129: container retaining protrusion

- 130: needle packaging
- 131: double-ended needle
- 131a: first end of needle 131
- 131b: second end of needle 131
- 132: packaging main body
- 133a: first end cap member
- 133b: second end cap member
- 134a: first frangible portion
- 134b: second frangible portion
- 135: protrusion
- 136: recess

- AD: axial direction
- CL: centre line
- h1: height of centre line CL over bottom of vial cavity 11
- h2: height of contact region 80 over bottom of vial cavity 11

## Claims

1. An apparatus for combining a first liquid component (90) stored in a container (125) and a second solid or liquid component stored in a vial (7) by means of negative pressure, comprising
a tray (1) having a vial cavity (11) for accommodating at least a portion of the vial (7) and a container cavity (12) for accommodating at least a portion of the container (125), and
a needle (131) having a first needle tip (131a) and a second needle tip (131b) for establishing a fluid communication between the vial (7) and the container (125) in a transfer position, in which the first needle tip (131a) is engaged with the vial (7) and the second needle tip (131b) is engaged with the container (125); wherein
the needle (131) is fixedly held at the tray (1) at an intermediate needle holding portion (13) between the vial cavity (11) and the container cavity (12), wherein
the vial cavity (11) is configured for guiding a movement of the vial (7) along an axial direction (AD) from an intermediate position, in which the first needle tip (131a) is not engaged with the vial (7), towards the transfer position, and
the container cavity (12) is configured for guiding a movement of the container (125) along the axial direction (AD) from an intermediate position, in which the second needle tip (131b) is not engaged with the container (125), towards the transfer position; and wherein
the tray (1) is having a planar upper surface, wherein
the vial cavity (11) and the container cavity (12) is each open toward the planar upper surface and each formed for fully accommodating the vial (7) and the container (125), respectively, in the intermediate position.

2. The apparatus as claimed in claim 1, wherein
the vial cavity (11) or the vial cavity (11) and the container cavity (12) each comprises retaining members (35a-35c, 36a, 36b; 128, 129), which are configured to position the vial (7) and the container (125), respectively, in the intermediate position and in parallel with the axial direction (AD).

3. The apparatus as claimed in claim 2, wherein
the retaining members (36a, 36b; 129) are further configured to guide an axial movement of the vial (7) and/or container (125) from the intermediate position to the transfer position while maintaining an axial alignment of the vial (7) and/or container (125) with the needle (131).

4. The apparatus as claimed in claim 3, wherein the retaining members comprise pairs of protrusions (36a, 36b; 129) formed on opposite side-walls (18, 121) of the vial cavity (11) and container cavity (12), respectively, which are configured for contacting side-surfaces of the vial (7) and container (125), respectively, for positioning the vial (7) and the container (125), respectively.

5. The apparatus as claimed in claim 4, wherein a height (h2) of contact regions (80) of the protrusions (36a, 36b; 129) with the side-surfaces of the vial (7) and container (125), respectively, above a bottom (17, 120) of the vial cavity (11) and container cavity (12), respectively, is larger than the height of a center line (CL) of the vial (7) and container (125) above the bottom (17, 120) of the vial cavity (11) and container cavity (12), respectively,
wherein the opposite side-walls (18, 121) on which the protrusions (36a, 36b; 129) are formed preferably are each upright and planar side-walls.

6. The apparatus as claimed in claim 4 or 5, wherein the vial cavity (11) comprises at least two pairs of protrusions (36a, 36b) formed on opposite side-walls (18) of the vial cavity (11), and at least one pair of protrusions (36b) is still in contact with side-surfaces of the vial (7) in the transfer position.

7. The apparatus as claimed in any of the preceding claims, wherein the vial cavity (11) further comprises axial position limiting members (35a, 35b) configured for delimiting an axial movement of the vial (7) inside the vial cavity (11) in a storage position,
wherein the axial position limiting members (35a, 35b) preferably are more flexible than the retaining members (36a, 36b) of the vial cavity (11).

8. The apparatus as claimed in any of the preceding claims, wherein the vial cavity (11) and the container cavity (12) each comprises a stop surface (19, 122a) for delimiting an axial displacement of the vial (7) and of the container (125) towards the needle (131) by abutment with a front end (77) of the vial (7) and a surface (127) of the container (125), respectively.

9. The apparatus as claimed in any of the preceding claims, wherein the vial cavity (11) further comprises a portion (15) that is sufficiently wide to enable access to a vial body (70) of the vial (7) by means of fingers of a user or grippers of a robot in the intermediate position for driving the axial displacement of the vial (7) towards the transfer position and/or for removal of the vial (7) in the intermediate position from the tray (1).

10. The apparatus as claimed in any of the preceding claims, wherein the vial cavity (11) further comprises a rear end cavity (14), where a bottom (71) of the vial (7) is sufficiently exposed to enable access to the bottom (71) for a finger of a user or a manipulation member of a robot for driving the axial movement of the vial (7) from the intermediate position to the transfer position.

11. The apparatus as claimed in any of the preceding claims,
wherein the tray (1) comprises a first tray member (10) comprising the vial cavity (11) configured for long-time storage of the vial (7), preferably under sterile conditions, and a second tray member (10') comprising the container cavity (125) configured for long-time storage of the container (125), preferably under sterile conditions.

12. The apparatus as claimed in any of the preceding claims, wherein
a central portion of the needle (131) is accommodated in a needle packaging (130) that is fixedly held at the tray (1) at the intermediate needle holding portion (13),
the needle tips (131a, 131b) are covered by cap members (133a, 133b) so that the needle (131) is packaged under sterile conditions,
the needle tips (131a, 131b) covered by the cap members (133a, 133b) protrude into the vial cavity (11) and container cavity (12), respectively, and
the cap members (133a, 133b) can be removed from the needle packaging (130) or opened for access to the needle tips (131a, 131b) of the needle (131) for fluid transfer.

13. The apparatus as claimed in claim 12, wherein the needle packaging (130) is held at the tray (1) at the intermediate needle holding portion (13) in a positive-fit manner.

14. A system for combining a first liquid component (90) stored in a container (125) and a second solid or liquid component stored in a vial (7) by means of negative pressure, comprising
the apparatus as claimed in any of the preceding claims, wherein
the vial (7) is accommodated in the vial cavity (11) of the tray (1) at an intermediate position, in which the first needle tip (131a) is not engaged with the vial (7), and
the container (125) is accommodated in the container cavity (12) at an intermediate position, in which the second needle tip (131b) is not engaged with the container (125).

15. A packaging unit (9) for packaging a vial (7) storing a second solid or liquid component together with a container (125) storing a first liquid component (90), comprising
a system as claimed in claim 14, and
a packaging foil (8), wherein
the tray (1) or at least the vial cavity (11) is sealed against the environment by the packaging foil (8).

## Patentansprüche

1. Vorrichtung zum Mischen einer ersten flüssigen Komponente (90), die in einem Behälter (125) aufbewahrt ist, und einer zweiten festen oder flüssigen Komponente, die in einem Fläschchen (7) aufbewahrt ist, mit Hilfe von Unterdruck, umfassend
ein Tablett (1) mit einer Fläschchen-Vertiefung (11) zur Aufnahme zumindest eines Abschnitts des Fläschchens (7) und einer Behälter-Vertiefung (12) zur Aufnahme zumindest eines Abschnitts des Behälters (125), und
eine Nadel (131) mit einer ersten Nadelspitze (131a) und einer zweiten Nadelspitze (131b) zum Herstellen einer Fluidverbindung zwischen dem Fläschchen (7) und dem Behälter (125) in einer Transferposition, in der die erste Nadelspitze (131a) mit dem Fläschchen (7) und die zweite Nadelspitze (131b) mit dem Behälter (125) in Eingriff steht; wobei
die Nadel (131) fest an dem Tablett (1) an einem Zwischen-Nadelhalteabschnitt (13) zwischen der Fläschchen-Vertiefung (11) und der Behälter-Vertiefung (12) gehalten ist, wobei
die Fläschchen-Vertiefung (11) so konfiguriert ist, dass diese eine Bewegung des Fläschchens (7) entlang einer axialen Richtung (AD) von einer Zwischenposition, in der die erste Nadelspitze (131a) nicht mit dem Fläschchen (7) in Eingriff steht, in Richtung der Transferposition führt, und
die Behälter-Vertiefung (12) so konfiguriert ist, dass diese eine Bewegung des Behälters (125) entlang der axialen Richtung (AD) von einer Zwischenposition, in der die zweite Nadelspitze (131b) nicht mit dem Behälter (125) in Eingriff steht, in Richtung der Transferposition führt;
und wobei
das Tablett (1) eine ebene Oberseite aufweist, wobei
die Fläschchen-Vertiefung (11) und die Behälter-Vertiefung (12) jeweils zu der ebenen Oberseite hin geöffnet sind und jeweils so ausgebildet sind, dass diese das Fläschchen (7) bzw. den Behälter (125) in der Zwischenposition vollständig aufnehmen können.

2. Vorrichtung nach Anspruch 1, wobei
die Fläschchen-Vertiefung (11) oder die Fläschchen-Vertiefung (11) und die Behälter-Vertiefung (12) jeweils Sicherungselemente (35a-35c, 36a, 36b; 128, 129) umfassen, die so gestaltet sind, dass diese das Fläschchen (7) bzw. den Behälter (125) in der Zwischenposition und parallel zur axialen Richtung (AD) positionieren.

3. Vorrichtung nach Anspruch 2, wobei
die Sicherungselemente (36a, 36b; 129) ferner so gestaltet sind, dass diese eine axiale Bewegung des Fläschchens (7) und/oder des Behälters (125) von der Zwischenposition in die Transferposition führen, während eine axiale Ausrichtung des Fläschchens (7) und/oder des Behälters (125) mit der Nadel (131) beibehalten wird.

4. Vorrichtung nach Anspruch 3, wobei die Sicherungselemente Paare von Vorsprüngen (36a, 36b; 129) umfassen, die an einander gegenüberliegenden Seitenwänden (18, 121) der Fläschchen-Vertiefung (11) bzw. der Behälter-Vertiefung (12) ausgebildet sind, die so gestaltet sind, dass diese Seitenflächen des Fläschchens (7) bzw. des Behälters (125) berühren, um das Fläschchen (7) bzw. den Behälter (125) zu positionieren.

5. Vorrichtung nach Anspruch 4, wobei eine Höhe (h2) von Kontaktbereichen (80) der Vorsprünge (36a, 36b; 129) mit den Seitenflächen des Fläschchens (7) bzw. des Behälters (125) über einem Boden (17, 120) der Fläschchen-Vertiefung (11) bzw. der Behälter-Vertiefung (12) größer ist als die Höhe einer Mittellinie (CL) des Fläschchens (7) und des Behälters (125) über dem Boden (17, 120) der Fläschchen-Vertiefung (11) bzw. der Behälter-Vertiefung (12),
wobei die einander gegenüberliegenden Seitenwände (18, 121), an denen die Vorsprünge (36a, 36b; 129) ausgebildet sind, vorzugsweise jeweils aufrechte und ebene Seitenwände sind.

6. Vorrichtung nach Anspruch 4 oder 5, wobei die Fläschchen-Vertiefung (11) zumindest zwei Paare von Vorsprüngen (36a, 36b) aufweist, die an einander gegenüberliegenden Seitenwänden (18) der Fläschchen-Vertiefung (11) ausgebildet sind, und zumindest ein Paar von Vorsprüngen (36b) in der Transferposition noch in Kontakt mit Seitenflächen des Fläschchens (7) ist.

7. Vorrichtung nach einem der vorhergehenden Ansprüche, wobei die Fläschchen-Vertiefung (11) ferner Axialpositions-Begrenzungselemente (35a, 35b) umfasst, die so gestaltet sind, dass diese eine axiale Bewegung des Fläschchens (7) innerhalb der Fläschchen-Vertiefung (11) in einer Aufbewahrungsposition begrenzen,
wobei die Axialpositions-Begrenzungselemente (35a, 35b) vorzugsweise flexibler sind als die Sicherungselemente (36a, 36b) der Fläschchen-Vertiefung (11).

8. Vorrichtung nach einem der vorhergehenden Ansprüche, wobei die Fläschchen-Vertiefung (11) und die Behälter-Vertiefung (12) jeweils eine Anschlagfläche (19, 122a) zur Begrenzung einer axialen Verschiebung des Fläschchens (7) und des Behälters (125) in Richtung der Nadel (131) durch Anlage an einem vorderen Ende (77) des Fläschchens (7) bzw. einer Oberfläche (127) des Behälters (125) aufweisen.

9. Vorrichtung nach einem der vorhergehenden Ansprüche, wobei die Fläschchen-Vertiefung (11) ferner einen Abschnitt (15) aufweist, der ausreichend breit ist, um einen Zugang zu einem Fläschchen-Körper (70) des Fläschchens (7) mittels der Finger eines Benutzers oder mittels Greifmitteln eines Roboters in der Zwischenposition zu ermöglichen, um die axiale Verstellung des Fläschchens (7) in Richtung der Transferposition zu bewirken und/oder um das Fläschchen (7) in der Zwischenposition aus dem Tablett (1) zu entfernen.

10. Vorrichtung nach einem der vorangehenden Ansprüche, wobei die Fläschchen-Vertiefung (11) ferner eine hintere End-Vertiefung (14) umfasst, in der ein Boden (71) des Fläschchens (7) ausreichend freiliegt, um einem Finger eines Benutzers oder einem Manipulationselement eines Roboters den Zugang zu dem Boden (71) zu ermöglichen, um die axiale Bewegung des Fläschchens (7) von der Zwischenposition in die Transferposition anzutreiben.

11. Vorrichtung nach einem der vorhergehenden Ansprüche,
wobei das Tablett (1) ein erstes Tablett-Element (10) mit der Fläschchen-Vertiefung (11), das für eine Aufbewahrung des Fläschchens (7) über einen längeren Zeitraum, vorzugsweise unter sterilen Bedingungen, ausgelegt ist, und ein zweites Tablett-Element (10') mit der Behälter-Vertiefung (125) aufweist, das für eine Aufbewahrung des Behälters (125) über einen längeren Zeitraum, vorzugsweise unter sterilen Bedingungen, ausgelegt ist.

12. Vorrichtung nach einem der vorhergehenden Ansprüche, wobei
ein zentraler Abschnitt der Nadel (131) in einer Nadelverpackung (130) aufgenommen ist, die fest an dem Tablett (1) an dem Zwischen-Nadelhalteabschnitt (13) gehalten ist,
die Nadelspitzen (131a, 131b) durch Kappenelemente (133a, 133b) abgedeckt sind, so dass die Nadel (131) unter sterilen Bedingungen verpackt ist,
die von den Kappenelementen (133a, 133b) abgedeckten Nadelspitzen (131a, 131b) in die Fläschchen-Vertiefung (11) bzw. die Behälter-Vertiefung (12) hineinragen, und
die Kappenelemente (133a, 133b) für einen Zugang zu den Nadelspitzen (131a, 131b) der Nadel (131) für den Flüssigkeitstransfer von der Nadelverpackung (130) entfernt werden können oder diese geöffnet werden kann.

13. Vorrichtung nach Anspruch 12, wobei die Nadelverpackung (130) formschlüssig am Tablett (1) an dem Zwischen-Nadelhalteabschnitt (13) gehalten ist.

14. System zum Mischen einer ersten flüssigen Komponente (90), die in einem Behälter (125) aufbewahrt ist, und einer zweiten festen oder flüssigen Komponente, die in einem Fläschchen (7) aufbewahrt ist, mit Hilfe von Unterdruck, umfassend
die Vorrichtung nach einem der vorhergehenden Ansprüche, wobei
das Fläschchen (7) in der Fläschchen-Vertiefung (11) des Tabletts (1) in einer Zwischenposition aufgenommen ist, in der die erste Nadelspitze (131a) nicht mit dem Fläschchen (7) in Eingriff steht, und
der Behälter (125) in der Behälter-Vertiefung (12) in einer Zwischenposition aufgenommen ist, in der die zweite Nadelspitze (131b) nicht mit dem Behälter (125) in Eingriff steht.

15. Verpackungseinheit (9) zum Verpacken eines Fläschchens (7), das eine zweite feste oder flüssige Komponente aufbewahrt, gemeinsam mit einem Behälter (125), der eine erste flüssige Komponente (90) aufbewahrt, umfassend
ein System nach Anspruch 14, und
eine Verpackungsfolie (8), wobei
das Tablett (1) oder zumindest der Fläschchen-Hohlraum (11) durch die Verpackungsfolie (8) gegen die Umgebung abgedichtet ist.

## Revendications

1. Un appareil pour mélanger un premier composant liquide (90) stocké dans un récipient (125) et un second composant solide ou liquide stocké dans une fiole (7) au moyen d'une pression négative, comprenant
un plateau (1) ayant une cavité de fiole (11) pour loger au moins une partie de la fiole (7) et une cavité de récipient (12) pour loger au moins une partie du récipient (125), et
une aiguille (131) ayant une première pointe d'aiguille (131a) et une deuxième pointe d'aiguille (131b) pour établir une communication fluidique entre la fiole (7) et le récipient (125) dans une position de transfert, dans laquelle la première pointe d'aiguille (131a) est engagée avec la fiole (7) et la deuxième pointe d'aiguille (131b) est engagée avec le récipient (125) ; dans laquelle
l'aiguille (131) est maintenue de manière fixe sur le plateau (1) au niveau d'une partie intermédiaire de maintien de l'aiguille (13) entre la cavité de fiole (11) et la cavité de récipient (12), dans laquelle
la cavité de fiole (11) est configurée pour guider un mouvement de la fiole (7) le long d'une direction axiale (AD) à partir d'une position intermédiaire, dans laquelle la première pointe d'aiguille (131a) n'est pas engagée avec la fiole (7), vers la position de transfert, et
la cavité de récipient (12) est configurée pour guider un mouvement du récipient (125) le long de la direction axiale (AD) à partir d'une position intermédiaire, dans laquelle la deuxième pointe d'aiguille (131b) n'est pas engagée avec le récipient (125), vers la position de transfert ;
et dans lequel
le plateau (1) présente une surface supérieure plane, dans laquelle
la cavité de fiole (11) et la cavité de récipient (12) sont chacune ouvertes vers la surface supérieure plane et chacune formée pour loger complètement la fiole (7) et le récipient (125), respectivement, dans la position intermédiaire.

2. L'appareil selon la revendication 1, dans lequel
la cavité de fiole (11) ou la cavité de fiole (11) et la cavité de récipient (12) comprennent chacune des éléments de retenue (35a-35c, 36a, 36b ; 128, 129), qui sont configurés pour positionner la fiole (7) et le récipient (125), respectivement, dans la position intermédiaire et parallèlement à la direction axiale (AD).

3. L'appareil selon la revendication 2, dans lequel
les éléments de retenue (36a, 36b ; 129) sont en outre configurés pour guider un mouvement axial de la fiole (7) et/ou du récipient (125) de la position intermédiaire à la position de transfert tout en maintenant un alignement axial de la fiole (7) et/ou du récipient (125) avec l'aiguille (131).

4. L'appareil selon la revendication 3, dans lequel les éléments de retenue comprennent des paires de saillies (36a, 36b ; 129) formées sur les parois latérales opposées (18, 121) de la cavité de fiole (11) et de la cavité de récipient (12), respectivement, qui sont configurées pour entrer en contact avec les surfaces latérales de la fiole (7) et du récipient (125), respectivement, afin de positionner la fiole (7) et le récipient (125), respectivement.

5. L'appareil selon la revendication 4, dans lequel une hauteur (h2) des zones de contact (80) des saillies (36a, 36b ; 129) avec les surfaces latérales de la fiole (7) et du récipient (125), respectivement, au-dessus d'un fond (17, 120) de la cavité de fiole (11) et de la cavité de récipient (12), respectivement, est supérieure à la hauteur d'une ligne centrale (CL) de la fiole (7) et du récipient (125) au-dessus du fond (17, 120) de la cavité de fiole (11) et de la cavité de récipient (12), respectivement,
les parois latérales opposées (18, 121) sur lesquelles sont formées les saillies (36a, 36b ; 129) sont de préférence des parois latérales droites et planes.

6. L'appareil selon la revendication 4 ou 5, dans lequel la cavité de fiole (11) comprend au moins deux paires de saillies (36a, 36b) formées sur les parois latérales opposées (18) de la cavité de fiole (11), et au moins une paire de saillies (36b) est toujours en contact avec les surfaces latérales de la fiole (7) dans la position de transfert.

7. L'appareil selon l'une des revendications précédentes, dans lequel la cavité de fiole (11) comprend en outre des éléments de limitation de la position axiale (35a, 35b) configurés pour délimiter un mouvement axial de la fiole (7) à l'intérieur de la cavité de fiole (11) dans une position de stockage,
les éléments de limitation de la position axiale (35a, 35b) étant de préférence plus souples que les éléments de retenue (36a, 36b) de la cavité de fiole (11).

8. L'appareil selon l'une quelconque des revendications précédentes, dans lequel la cavité de fiole (11) et la cavité de récipient (12) comprennent chacune une surface de butée (19, 122a) pour délimiter un déplacement axial du flacon (7) et du récipient (125) vers l'aiguille (131) par butée avec une extrémité avant (77) du flacon (7) et une surface (127) du récipient (125), respectivement.

9. L'appareil selon l'une quelconque des revendications précédentes, dans lequel la cavité de fiole (11) comprend en outre une partie (15) suffisamment large pour permettre l'accès à un corps de fiole (70) de la fiole (7) au moyen des doigts d'un utilisateur ou des pinces d'un robot dans la position intermédiaire pour entraîner le déplacement axial de la fiole (7) vers la position de transfert et/ou pour retirer la fiole (7) dans la position intermédiaire du plateau (1).

10. L'appareil selon l'une quelconque des revendications précédentes, dans lequel la cavité de fiole (11) comprend en outre une cavité arrière (14), où le fond (71) de la fiole (7) est suffisamment exposé pour permettre l'accès au fond (71) à un doigt d'un utilisateur ou à un organe de manipulation d'un robot afin d'entraîner le déplacement axial de la fiole (7) de la position intermédiaire vers la position de transfert.

11. L'appareil selon l'une des revendications précédentes,
dans lequel le plateau (1) comprend un premier élément de plateau (10) comprenant la cavité de fiole (11) configurée pour le stockage à long terme de la fiole (7), de préférence dans des conditions stériles, et un deuxième élément de plateau (10') comprenant la cavité de récipient (125) configurée pour le stockage à long terme du récipient (125), de préférence dans des conditions stériles.

12. L'appareil selon l'une quelconque des revendications précédentes, dans lequel
une partie centrale de l'aiguille (131) est logée dans un emballage d'aiguille (130) qui est maintenu de manière fixe sur le plateau (1) au niveau de la partie intermédiaire de maintien de l'aiguille (13),
les pointes de l'aiguille (131a, 131b) sont recouvertes par des éléments de capuchon (133a, 133b) afin que l'aiguille (131) soit emballée dans des conditions stériles,
les pointes d'aiguille (131a, 131b) recouvertes par les éléments de capuchon (133a, 133b) font saillie dans la cavité de fiole (11) et la cavité de récipient (12), respectivement, et
les éléments du capuchon (133a, 133b) peuvent être retirés de l'emballage de l'aiguille (130) ou ouverts pour accéder aux pointes (131a, 131b) de l'aiguille (131) pour le transfert de fluide.

13. L'appareil selon la revendication 12, dans lequel l'emballage de l'aiguille (130) est maintenu sur le plateau (1) au niveau de la partie intermédiaire de maintien de l'aiguille (13) de manière positive.

14. Un système permettant de combiner un premier composant liquide (90) stocké dans un récipient (125) et un second composant solide ou liquide stocké dans une fiole (7) au moyen d'une pression négative, comprenant
l'appareil tel que revendiqué dans l'une quelconque des revendications précédentes, dans lequel
la fiole (7) est logé dans la cavité de fiole (11) du plateau (1) dans une position intermédiaire, dans laquelle la première pointe d'aiguille (131a) n'est pas engagée avec la fiole (7), et
le récipient (125) est logé dans la cavité de récipient (12) à une position intermédiaire, dans laquelle la seconde pointe d'aiguille (131b) n'est pas engagée avec le récipient (125).

15. Une unité d'emballage (9) pour emballer une fiole (7) stockant un second composant solide ou liquide avec un récipient (125) stockant un premier composant liquide (90), comprenant
un système selon la revendication 14, et
une feuille d'emballage (8), dans laquelle
le plateau (1) ou au moins la cavité de fiole (11) est scellé contre l'environnement par la feuille d'emballage (8).
